(19) Europäisches Patentamt
European Patent Office
Office européen des brevets

(11) EP 0 710 641 B1

(12) # FASCICULE DE BREVET EUROPEEN

(45) Date de publication et mention de la délivrance du brevet:
**01.07.1998 Bulletin 1998/27**

(51) Int. Cl.$^6$: **C07C 43/12**, C07C 41/22

(21) Numéro de dépôt: **95402400.6**

(22) Date de dépôt: **26.10.1995**

(54) **Procédé de préparation de l'oxyde de 1,1-dichlorométhyle et de méthyle ou de l'oxyde de 1,1-dichlorométhyle et d'éthyle**

Verfahren zur Herstellung von (1,1-Dichloromethyl)methylether und (1,1-Dichloromethyl)ethylether

Process for the preparation of 1,1-dichloromethyl methyl ether and 1,1-dichloromethyl ethyl ether

(84) Etats contractants désignés:
**CH DE FR GB LI NL**

(30) Priorité: **04.11.1994 FR 9413210**

(43) Date de publication de la demande:
**08.05.1996 Bulletin 1996/19**

(73) Titulaire:
**SOCIETE NATIONALE DES POUDRES ET EXPLOSIFS**
**F-75181 Paris Cédex 04 (FR)**

(72) Inventeurs:
- **Dabee, Annick**
  **F-91760 Itteville (FR)**
- **Gauthier, Patricia**
  **F-91590 Cerny (FR)**
- **Senet, Jean-Pierre**
  **Herbeauvilliers, F-77760 Buthiers (FR)**

(74) Mandataire: **Pech, Bernard et al**
**Sté Nationale des Poudres et Explosifs**
**12, quai Henri IV**
**75181 Paris Cédex 04 (FR)**

(56) Documents cités:
**EP-A- 0 514 683** **DE-A- 1 443 704**

- **JOURNAL OF ORGANIC CHEMISTRY, vol. 23, no. 5, 22 Mai 1958 EASTON US, pages 745-746, L. R. EVANS ET AL 'Preparation of certain polychlorodimethyl ethers'**
- **'Organic Syntheses Volume 47' 1967 , JOHN WILEY , NEW YORK * page 47 - page 49 ***

EP 0 710 641 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.16.3/3.4

## Description

L'invention concerne un nouveau procédé de préparation de l'oxyde de 1,1-dichlorométhyle et de méthyle ($CH_3$-O-$CHCl_2$) ou de l'oxyde de 1,1-dichlorométhyle et d'éthyle ($C_2H_5$-O-$CHCl_2$).

Il est connu, d'après l'article paru dans J. Org. Chem. 1958, 23, pp 745-746, de préparer l'oxyde de 1,1-dichlorométhyle et de méthyle par chloration photochimique de l'oxyde de chlorométhyle et de méthyle.

Le schéma réactionnel est le suivant :

$$CH_3\text{-}O\text{-}CH_2Cl + Cl_2 \xrightarrow[-HCl]{} CH_3\text{-}O\text{-}CHCl_2 + ClCH_2\text{-}O\text{-}CH_2Cl$$

Ce procédé présente de graves inconvénients. L'oxyde de chlorométhyle et de méthyle utilisé comme matière de départ est un composé cancérigène. Le rendement de la réaction est très faible notamment du fait qu'il se forme également de l'oxyde de bis(chlorométhyle). Celui-ci est de plus très fortement cancérigène et sa présence dans un produit commercial est interdite.

L'obtention de l'oxyde de 1,1-dichlorométhyle et d'éthyle par ce procédé est encore plus difficile en raison de la chloration qui se produit sur le radical éthyle et qui donne naissance à plusieurs produits secondaires.

Ce procédé est par conséquent pratiquement inutilisable.

Un autre procédé a été décrit dans l'article paru dans Org. Synth. Coll. Vol. V, pp 365-367 et dans le résumé CA n° 55 : 18557 *f*. Il consiste à effectuer la chloration du formiate au moyen du pentachlorure de phosphore selon le schéma réactionnel suivant :

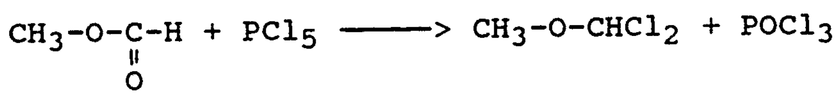

Mais ce procédé de laboratoire est difficilement transposable à l'échelon industriel. Le pentachlorure de phosphore est un solide très hygroscopique dont la manipulation est de ce fait dangereuse. Les grandes quantités d'effluents phosphorés qui se forment doivent être traitées. Les oxydes de 1,1-dichlorométhyle se décomposent rapidement en présence de grandes quantités de $PCl_5$ et/ou $POCl_3$, ce qui rend difficile leur isolement du milieu réactionnel. Ils sont de plus obtenus avec une pureté qui n'est pas satisfaisante, car ils contiennent une certaine quantité de composés phosphorés.

Il existait donc un besoin pour un procédé de préparation des oxydes de 1,1-dichlorométhyle qui ne présente pas les inconvénients des procédés antérieurs.

L'invention a pour objet un procédé de préparation de l'oxyde de 1,1-dichlorométhyle et de méthyle ou de l'oxyde de 1,1-dichlorométhyle et d'éthyle selon lequel on fait réagir respectivement le formiate de méthyle ou le formiate d'éthyle avec le phosgène, le diphosgène, le triphosgène ou le chlorure d'oxalyle, ou un de leurs mélanges, à une température comprise entre 40°C et 100°C, en présence d'un catalyseur choisi dans le groupe constitué par :

- les oxydes et les sulfures de phosphines trisubstitués de formule générale (I)

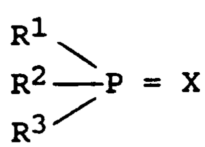

  dans laquelle X représente un atome d'oxygène ou de soufre, $R^1$, $R^2$ et $R^3$, identiques ou différents, représentent un radical aromatique substitué ou non,
- les dichlorures de phosphines trisubstitués de formule générale (II)

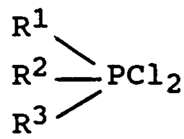

dans laquelle $R^1$, $R^2$ et $R^3$ ont la signification précitée,

- les formamides de formule générale (III)

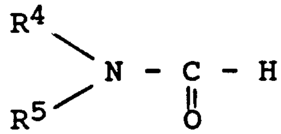

dans laquelle $R^4$ et $R^5$, identiques ou différents, représentent chacun un radical aliphatique en $C_4$ à $C_{10}$ ou un radical cyclohexyle,

- les produits de réaction des formamides de formule (III) avec les agents chlorurants,
- et leurs mélanges.

Le schéma réactionnel est le suivant :

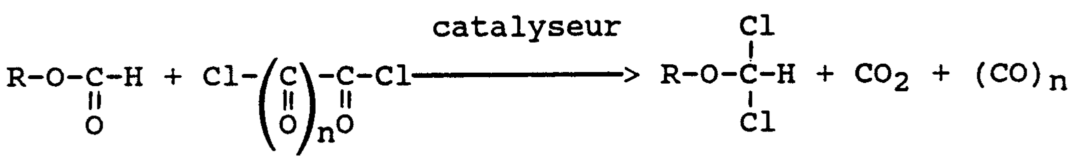


dans lequel R représente le groupe méthyle ou éthyle, et n représente le nombre O ou 1.

Le procédé selon l'invention permet la préparation industrielle, avec de bons rendements et une très grande pureté, de l'oxyde de 1,1-dichlorométhyle et de méthyle ou d'éthyle. Les matières premières sont bon marché. Sa mise en oeuvre est aisée. Le traitement des effluents est simple. Le procédé est par conséquent économique et les risques toxiques sont strictement limités à l'unité de production.

La présence d'un catalyseur est indispensable pour obtenir les oxydes de 1,1-dichlorométhyle. Sans catalyseur, dans les autres conditions de la réaction, on n'observe aucune réaction.

Les catalyseurs de formule (I) préférés sont ceux dans laquelle X représente un atome d'oxygène.

Les substituants des radicaux $R^1$, $R^2$ et $R^3$ sont en particulier choisis parmi les atomes d'halogène tels que de préférence le chlore, le brome ou le fluor, les radicaux alkyle en $C_1$ à $C_4$, alkoxy en $C_1$ à $C_4$ et le groupe nitro.

Les radicaux $R^1$, $R^2$ et $R^3$ sont de préférence identiques et de préférence représentent un radical phényle substitué ou non.

Comme exemples de catalyseurs de formule (I) utilisables selon l'invention, on peut citer notamment l'oxyde de triphénylphosphine, le sulfure de triphényl phosphine, l'oxyde de tri-p-tolylphosphine.

Le catalyseur préféré est l'oxyde de triphénylphosphine.

Les catalyseurs de formule (I) ou de formule (II) peuvent être fixés sur un polymère par l'intermédiaire d'un des radicaux $R^1$, $R^2$ et $R^3$. A titre d'exemple, on peut citer les polymères qui comportent un enchaînement :

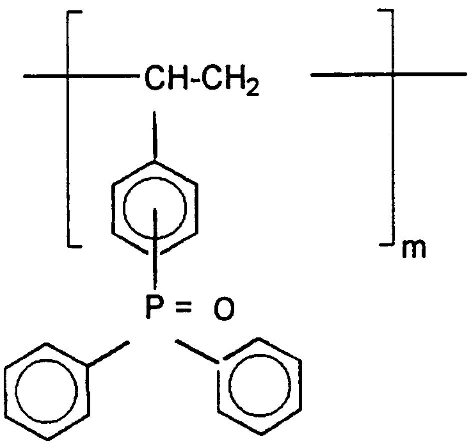

Les catalyseurs préférés de formule (III) sont les catalyseurs dans lesquels $R^4$ ou $R^5$ représente un radical butyle ou isobutyle ou un radical cyclohexyle. On peut citer en particulier le dibutyl- ou le diisobutylformamide et le dicyclohexylformamide.

Les produits de réaction des formamides de formule (III) avec des agents chlorurants sont obtenus de façon connue par réaction de ces formamides avec des agents chlorurants tels que par exemple de chlorure de thionyle, le chlorure d'oxalyle, le phosgène et l'oxychlorure de phosphore. De préférence, les formamides de formule (III) préférées qui sont mis à réagir sont celles dans lesquelles $R^4$ et $R^5$ représentent les radicaux mentionnés au paragraphe ci-dessus.

On peut également employer un mélange de plusieurs des catalyseurs précédemment décrits.

Généralement on ajoute le catalyseur en quantité comprise entre 5 et 50% et de préférence entre 10 et 40% en mole par rapport au formiate.

La température de la réaction peut être comprise entre 40° et 100°C. Pour l'obtention de l'oxyde de 1,1-dichlorométhyle et de méthyle, elle est de préférence comprise entre 60°C et 90°C.

La réaction est effectuée dans un milieu liquide. Pour ce faire, on opère à une pression suffisamment élevée pour que le formiate soit liquide ou on utilise un solvant organique inerte vis à vis des réactifs et dont le point d'ébullition est suffisamment élevé pour permettre d'obtenir les températures souhaitées dans le milieu réactionnel.

Comme solvants convenant bien, on peut citer le chlorobenzène, les dichlorobenzènes, les trichlorobenzènes et les xylènes. Le solvant préféré est l'o-dichlorobenzène.

Le composé qui est mis à réagir avec le formiate est ajouté au moins en quantité stoechiométrique et de préférence en excès par rapport au formiate. Le réactif préféré est le phosgène.

Pour la mise en oeuvre du procédé on utilise les dispositifs convenables connus.

Lorsque le réactif est le phosgène, un mode opératoire approprié est le suivant : le solvant, le catalyseur et le formiate sont introduits dans le réacteur. Le milieu réactionnel est chauffé à la température choisie puis on ajoute progressivement le phosgène gazeux dans le milieu réactionnel. La réaction est terminée au bout de plusieurs heures. L'oxyde de 1,1-dichlorométhyle est isolé par distillation et est obtenu avec une très grande pureté et un bon rendement.

Les applications des oxydes de 1,1-dichlorométhyle sont nombreuses. Une des plus importantes est la formylation des composés aromatiques ou hétéroaromatiques en présence d'un catalyseur de Friedel et Craft qui permet d'obtenir les aldéhydes correspondants avec d'excellents rendements et sans sous-produits gênants.

Les exemples qui suivent illustrent l'invention sans toutefois la limiter.

Exemple 1 :

Dans un réacteur équipé de dispositifs d'agitation et de prise de température, de condenseurs et de tubulures d'alimentation, on introduit, à température ambiante (aux environs de 20°C), 186 g d'o-dichlorobenzène, 22,23 g (0,08 mol) d'oxyde de triphénylphosphine (OTPP) puis 24,37 g (0,41 mol) de formiate de méthyle et on chauffe le milieu réactionnel à 68°-70°C. On introduit ensuite progressivement 53,4 g (0,54 mol) de phosgène gazeux pendant 7 heures en maintenant la température entre 70° et 80°C.

La réaction est suivie par analyse chromatographique en phase gazeuse (CPG) en utilisant un étalon (alcane en $C_8$). Lorsque la réaction est terminée, le taux de transformation du formiate de méthyle en oxyde de 1,1-dichlorométhyle et de méthyle (ODCMM) déterminée par CPG est de 95% et la quantité de formiate de méthyle non transformé, déterminée elle aussi par CPG est de 5%.

On purifie l'ODCMM par distillation sous pression réduite. On obtient 33,8 g (rendement 72%) d'ODCMM ayant une pureté de 100% (déterminée par analyse CPG).

Exemples 2 à 6 :

En opérant de manière analogue à celle de l'exemple 1, on a effectué des essais avec différents catalyseurs en faisant varier les proportions des constituants et les conditions opératoires. Ceux-ci et les résultats obtenus sont indiqués dans le tableau suivant :

| Exemples | Formiate de méthyle % en poids (a) | Catalyseur % en mole (b) | COCl2 % en mole (b) | Temps Heure | Température °C | % (c) par analyse CPG (d) | |
|---|---|---|---|---|---|---|---|
| | | | | | | Formiate non transformé | ODCMM formé |
| 2 | 13 | OTPP 10 | 186 | 2<br>16<br>21 | 60<br>60-93<br>80 | 6 | 76 |
| 3 | 11,5 | OTPP 20 | 147 | 6,75 | 70-75 | 10 | 90 |
| 4 | 23,2 | OTPP 19,7 | 134 | 4<br>4 | 60<br>70-75 | 5 | 95 |
| 5 | 13 | DBF 10,5 | 153 | 7,50<br>20,50 | 50<br>50-70 | 46 | 31 |
| 6 | 13 | TPPCl2 25 | 150 | 7,5 | 70-75 | - | 98 |

DBF : N,N-dibutylformamide
TPPCl2 : Dichlorure de triphénylphosphine
a) dans le mélange de formiate de méthyle et d'o-dichlorobenzène
b) par rapport au formiate de méthyle
c) par rapport à la quantité de formiate initiale
d) avec un étalon (alcane en C8)

Exemple 7 :

Dans un réacteur semblable à celui de l'exemple 1, on introduit 3,61 g de formiate de méthyle et le dichlorure de triphénylphosphine fixé sur une résine, préparé par chloration de 5,4 g de triphénylphosphine supporté sur des billes polystyréniques (3 mmol P/g) dans 26,5 g d'o-dichlorobenzène, puis on chauffe le milieu réactionnel à 68°C.

On introduit ensuite progressivement 7,5 g de phosgène pendant 2 heures en maintenant la température entre 68° et 80°C et on continue le chauffage à cette température pendant 9 heures. Le taux de transformation du formiate de méthyle en oxyde de 1,1-dichlorométhyle et de méthyle déterminée par CPG est de 27%.

Exemple 8 :

Dans un réacteur semblable à celui de l'exemple 1, on introduit 25,85 g (0,35 mol) de formiate d'éthyle, 160 g d'o-dichlorobenzène et 24,35 g (0,087 mol) d'oxyde de triphénylphosphine puis on chauffe le milieu réactionnel aux environs de 71°C. On introduit ensuite progressivement 50 g (0,505 mol) de phosgène pendant 7 heures et demi à une température d'environ 75°C puis on poursuit le chauffage à cette température pendant environ 3 heures.

On refroidit le milieu vers 25°C, on ajoute 30 g de chloroforme, on abaisse la température à - 2°C et on enlève par filtration le complexe formé par le catalyseur et le chloroforme.

L'analyse CPG du filtrat permet de déterminer que le taux de transformation du formiate d'éthyle en oxyde de 1,1-dichlorométhyle et d'éthyle est de 88% et la quantité de formiate d'éthyle non transformée est de 6%.

On distille le filtrat sous pression réduite, (Point d'ébullition : Eb = 35°-38°C/65-70 mm Hg et Eb = 56°-65°C/350 mm Hg). On obtient ainsi l'oxyde de 1,1-dichlorométhyle et d'éthyle avec un rendement de 58%.

**Revendications**

**1.** Procédé de préparation de l'oxyde de 1,1-dichlorométhyle et de méthyle ou de l'oxyde de 1,1-dichlorométhyle et d'éthyle, caractérisé en ce qu'on fait réagir le formiate de méthyle ou le formiate d'éthyle avec le phosgène, le diphosgène, le triphosgène ou le chlorure d'oxalyle, ou un de leurs mélanges, à une température comprise entre 40°C et 100°C, en milieu liquide, en présence d'un catalyseur choisi dans le groupe constitué par :

- les oxydes et les sulfures de phosphines trisubstitués de formule générale (I)

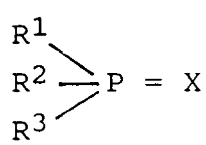

dans laquelle X représente un atome d'oxygène ou de soufre, $R^1$ $R^2$ et $R^3$, identiques ou différents, représentent un radical aromatique substitué ou non,

- les dichlorures de phosphines trisubstitués de formule générale (II)

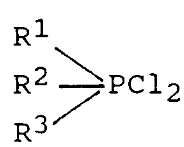

dans laquelle $R^1$, $R^2$ et $R^3$ ont la signification précitée,

- les formamides de formule générale (III)

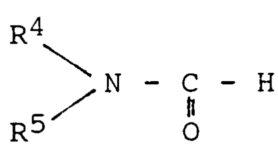

dans laquelle $R^4$ et $R^5$, identiques ou différents, représentent chacun un radical aliphatique en $C_4$ à $C_{10}$ ou un

radical cyclohexyle,

- les produits de réaction des formamides de formule (III) avec les agents chlorurants,
- et leurs mélanges.

**2.** Procédé selon la revendication 1, caractérisé en ce que l'on fait réagir le phosgène avec le formiate.

**3.** Procédé selon la revendication 1, caractérisé en ce que l'on utilise un solvant organique, inerte vis à vis des réactifs, choisi parmi le chlorobenzène, les dichlorobenzènes, les trichlorobenzènes et les xylènes.

**4.** Procédé selon la revendication 3, caractérisé en ce que le solvant est l'o-dichlorobenzène.

**5.** Procédé selon la revendication 1, caractérisé en ce que dans la formule générale (I), X représente l'oxygène.

**6.** Procédé selon la revendication 1, caractérisé en ce que les substituants des radicaux $R^1$, $R^2$ et $R^3$ sont choisis dans le groupe constitué par les atomes d'halogène, les radicaux alkyle en $C_1$ à $C_4$, alkoxy en $C_1$ à $C_4$ et le groupe nitro.

**7.** Procédé selon la revendication 1, caractérisé en ce que les catalyseurs de formule (I) ou de formule (II) sont fixés sur un polymère.

**8.** Procédé selon la revendication 1, caractérisé en ce que le catalyseur est l'oxyde de triphénylphosphine.

**9.** Procédé selon la revendication 1, caractérisé en ce que $R^4$ et $R^5$, identiques ou différents, représentent le radical butyle ou isobutyle.

**10.** Procédé selon la revendication 1, caractérisé en ce que la quantité de catalyseur est comprise entre 5 et 50% en mole par rapport au formiate.

**11.** Procédé selon la revendication 1, caractérisé en ce que le composé qui est mis à réagir avec le formiate est ajouté en quantité stoechiométrique ou en excès par rapport au formiate.

## Claims

**1.** Process for the preparation of 1,1-dichloromethyl methyl ether or of 1,1-dichloromethyl ethyl ether, characterised in that methyl formate or ethyl formate is reacted with phosgene, diphosgene, triphosgene or oxalyl chloride or a mixture thereof at a temperature of between 40°C and 100°C in a liquid medium in the presence of a catalyst selected from the group comprising:

- oxides and sulphides of trisubstituted phosphines of the general formula (I)

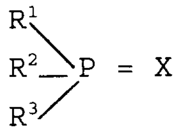

in which X stands for an oxygen or sulphur atom, $R^1$, $R^2$ and $R^3$ may be the same or different, and stand for an aromatic radical which may or may not be substituted,

- dichlorides of trisubstituted phosphines of the general formula (II)

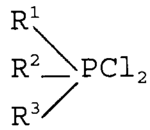

in which $R^1$, $R^2$ and $R^3$ denote the same as above,

- formamides of the general formula (III)

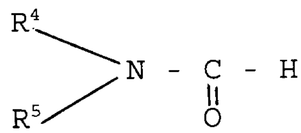

in which $R^4$ and $R^5$ may be the same or different, and each stand for a $C_4$-$C_{10}$ aliphatic radical or a cyclohexyl radical,

- reaction products of formamides of the formula (III) with chlorinating agents,

- and mixtures thereof.

**2.** Process according to Claim 1, characterised in that phosgene is reacted with the formate.

**3.** Process according to Claim 1, characterised in that an organic solvent is used which is inert to the reagents and is selected from among chlorobenzene, dichlorobenzenes, trichlorobenzenes and xylenes.

**4.** Process according to Claim 3, characterised in that the solvent is o-dichlorobenzene.

**5.** Process according to Claim 1, characterised in that X stands for oxygen in the general formula (I).

**6.** Process according to Claim 1, characterised in that the substituents on the radicals $R^1$, $R^2$ and $R^3$ are selected from the group comprising halogen atoms, $C_1$-$C_4$ alkyl radicals, $C_1$-$C_4$ alkoxy radicals and the nitro group.

**7.** Process according to Claim 1, characterised in that the catalysts of the formula (I) or of the formula (II) are fixed on a polymer.

**8.** Process according to Claim 1, characterised in that the catalyst is triphenylphosphine oxide.

**9.** Process according to Claim 1, characterised in that $R^4$ and $R^5$ may be the same or different, and stand for the butyl or isobutyl radical.

**10.** Process according to Claim 1, characterised in that the quantity of catalyst is between 5 and 50 mol.%, with reference to the formate.

**11.** Process according to Claim 1, characterised in that the compound which is reacted with the formate is added in a stoichiometric quantity or in an excess, with reference to the formate.

**Patentansprüche**

**1.** Verfahren zur Herstellung von (1,1-Dichlormethyl)-methylether oder von (1,1-Dichlormethyl)-ethylether, dadurch gekennzeichnet, daß man Ameisensäuremethylester oder Ameisensäureethylester mit Phosgen, Diphosgen, Triphosgen oder Oxalylchlorid oder einem Gemisch davon bei einer Temperatur von 40 bis 100°C in Gegenwart eines Katalysators, der aus folgender Gruppe ausgewählt ist, in flüssigem Medium umsetzt:

- trisubstituierte Oxide und Sulfide von Phosphinen der allgemeinen Formel (I)

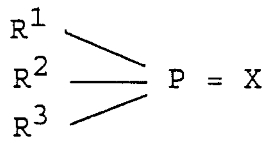

in der X ein Sauerstoff- oder Schwefelatom bedeutet, $R^1$, $R^2$ und $R^3$ gleich oder verschieden sind und einen gegebenenfalls substituierten aromatischen Rest bedeuten,

- trisubstituierte Dichlorphosphine der allgemeinen Formel (II)

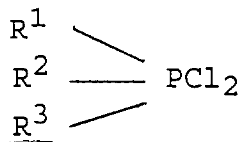

in der $R^1$, $R^2$ und $R^3$ die vorerwähnten Bedeutungen haben,

- Formamide der allgemeinen Formel (III)

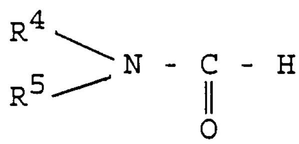

in der $R^4$ und $R^5$ gleich oder verschieden sind und jeweils einen aliphatischen $C_4$-$C_{10}$-Rest oder einen Cyclohexylrest bedeuten,

- Reaktionsprodukte aus Formamiden der Formel (III) mit Chlorierungsmitteln und
- Gemische davon.

**2.** Verfahren nach Anspruch 1, dadurch gekennzeichnet, daß man Phosgen mit dem Ameisensäureester umsetzt.

**3.** Verfahren nach Anspruch 1, dadurch gekennzeichnet, daß man ein gegenüber den Reaktanten inertes organisches Lö-Lösungsmittel verwendet, das unter Chlorbenzol, Dichlorbenzolen, Trichlorbenzolen und Xylolen ausgewählt ist.

**4.** Verfahren nach Anspruch 3, dadurch gekennzeichnet, daß es sich beim Lösungsmittel um o-Dichlorbenzol handelt.

**5.** Verfahren nach Anspruch 1, dadurch gekennzeichnet, daß in der allgemeinen Formel (I) X die Bedeutung Sauerstoff hat.

**6.** Verfahren nach Anspruch 1, dadurch gekennzeichnet, daß die Substituenten der Reste $R^1$, $R^2$ und $R^3$ unter Halogenatomen, $C_1$-$C_4$-Alkylresten, $C_1$-$C_4$-Alkoxyresten und der Nitrogruppe ausgewählt werden.

**7.** Verfahren nach Anspruch 1, dadurch gekennzeichnet, daß die Katalysatoren der Formel (I) oder der Formel (II) an einem Polymeren fixiert sind.

**8.** Verfahren nach Anspruch 1, dadurch gekennzeichnet, daß es sich beim Katalysator um Triphenylphosphinoxid handelt.

**9.** Verfahren nach Anspruch 1, dadurch gekennzeichnet, daß $R^4$ und $R^5$, die gleich oder verschieden sind, Butyl- oder Isobutylreste bedeuten.

**10.** Verfahren nach Anspruch 1, dadurch gekennzeichnet, daß die Menge des Katalysators 5 bis 50 Mol-%, bezogen auf den Ameisensäureester, beträgt.

11. Verfahren nach Anspruch 1, dadurch gekennzeichnet, daß die Verbindung, die mit dem Ameisensäureester umgesetzt wird, im Verhältnis zum Ameisensäureester in einer stöchiometrischen Menge oder im Überschuß zugesetzt wird.